# EUROPEAN PATENT SPECIFICATION

(11) **EP 3 586 901 B1**
(45) Date of publication and mention of the grant of the patent: **02.03.2022**
(21) Application number: 19183481.1
(22) Date of filing: 28.10.2009
(51) Int. Cl.: A61M 29/00, A61M 25/00, A61M 25/09

(54) **CATHETERISATION DEVICE AND KIT**
EINFÜHRUNGSHILFE EINES KATHETERS UND KIT
DISPOSITIF DE CATHÉTÉRISATION ET KIT

(30) Priority: 31.10.2008 GB 0820052
(43) Date of publication of application: 01.01.2020
(62) Divisional of application: 09760281.7
(73) Proprietor: UrethroTech Ltd, Kingston upon Thames, Surrey KT2 5NY (GB)
(72) Inventor: ANDRICH, Daniela, Kingston upon Thames,, Surrey, KT2 5NY (GB)
(74) Representative: Kehoe, Laura Ellen

(56) References cited:
- FR-A- 2 633 835
- US-A- 4 938 746
- US-A- 4 942 869
- US-A- 5 205 830
- US-A- 5 391 155
- US-A1- 2007 185 522

## Description

### FIELD

The present invention relates to a device and a related kit for urethral stricture dilatation.

### BACKGROUND

Catheterisation involves the insertion of a catheter into a body cavity, duct or vessel, thereby allowing drainage or injection of fluids or access by surgical instruments. In the case of urinary catheterisation, a catheter is inserted into a patient's bladder usually for the treatment of urinary incontinence, or to release urine from the bladder when the patient is in acute urinary retention due to, for example, prostate enlargement or post-operatively to monitor urine output when a patient is ill and unable to move. The catheter is normally inserted through the patient's urethra, but if the urethra is blocked or damaged the catheter may need to be inserted into the patient's bladder through the wall of the abdomen instead.

There are two main types of urinary catheterisation. Indwelling catheterisation means that a catheter is left in the body for a period of time. It should be performed by a medical practitioner. Intermittent catheterisation is the temporary placement of the catheter to empty the patient's bladder and may be performed by a medical practitioner or by the patient (in which case it is termed intermittent self-catheterisation).

Catheters for indwelling catheterisation typically have two or three channels and are retained inside the bladder by a balloon located at the tip of the catheter. The balloon is inflated by pumping air, water or saline solution down the narrow balloon channel of the catheter using a syringe. Once inflated, the balloon keeps the catheter securely in the bladder while the primary channel of the catheter allows urinary drainage. An optional third irrigation channel may be used to wash away blood and small clots that may be present after bladder or prostate surgery. This prevents larger clots from forming, which might otherwise block the catheter.

Catheters for intermittent catheterisation do not have a balloon at the tip because they are not intended to be retained in the bladder.

Where a patient has a blocked urine flow due to a narrowing of the urethra, known as a urethral stricture, the stricture will often need to be stretched before a catheter can be inserted. Stretching of the stricture can be achieved using a urethral dilator.

A problem with known types of catheters and dilators, is that correct insertion of the device can be a difficult and potentially harmful procedure due to the natural curvature of the bulbar urethra in men. It can be difficult to advance the catheter/dilator into the part of the urethra known as the prostatic urethra which curves upwards towards the bladder, especially when the prostate is enlarged. The distal tip can cause trauma to the urethral tissue as the user attempts to navigate the catheter along the curved path of the bulbar urethra towards the prostatic urethra. Tissue trauma can lead to the formation of a false passage (i.e. a hole dug into the wall of the urethra), which can make future urethral catheterisation attempts difficult, if not impossible.

If malposition of the catheter is not noticed, inflation of the catheter balloon whilst the tip of the catheter is still in the urethra (a risk associated with known types of indwelling catheters) can seriously damage the urethral tissue, causing pain, bleeding and traumatic rupture. The rupture can heal with a urethral stricture which may require dilatation or surgery at a later date. At worst, if the urethral tissue damage is not noticed in time, subsequent urethral ischaemic necrosis can result in associated life threatening sepsis and loss of urethral and penile tissue.

There is a need to improve upon current urethral stricture dilatation devices and kits in order to improve the safety of the procedures, to simplify and speed up insertion and to improve the performance of the devices.

US5391155 A describes an arrangement for injection of X-ray contrast medium comprising a ureter tube, an auxiliary tube and a mandrin. US4938746 describes a nasogastric device comprising a removable stylet or stiffening wire for easing insertion.

### SUMMARY

Described herein, but not forming part of the invention, is a device for urinary catheterisation comprising:
a catheter having an elongate body provided with a distal tip, a proximal end and one or more channels extending therethrough;
a guidewire extending through one of said one or more channels for guiding the catheter upon insertion; and
a lubrication port at or towards the proximal end of the elongate body for introduction of a guidewire lubrication fluid into the channel having the guidewire extending therethrough.

Preferably, the guidewire is provided with a stopper in order to prevent the proximal end of the guidewire from passing through the guidewire channel towards the distal tip of the catheter and becoming lost in the urethra or bladder. The stopper may be adapted to receive a syringe for introducing guidewire lubrication fluid into the guidewire channel when the stopper is coupled to the lubrication port. Lubrication of the guidewire is important since it reduces friction between the guidewire and the urethra and also between the guidewire and the catheter.

The stopper may take various forms, for example the stopper may comprise a head portion and a body portion arranged in a T-shaped configuration and having a hollow core extending therethrough, with the body portion capable of being received in the lubrication port and the head portion capable of receiving the syringe. Alternatively, the stopper may comprise only a head portion having a hollow core extending therethrough, the head portion capable of receiving the syringe. In each configuration of stopper the head portion may suitably be larger than the opening of the lubrication port. The stopper may be releasably connected to the lubrication port, preferably by a screw means, a push-fit means or a twist-lock means, though other mechanisms such as a snap-lock or a Luer Lock may equally be used.

Moreover, the stopper may be provided with an anchor for connecting the proximal end of the guidewire to the stopper. The anchor may be suitably configured so as to allow the guidewire lubrication fluid to flow around the anchor as it passes through the stopper and into the guidewire channel. Preferably, the anchor comprises a band, which traverses the width of the hollow core of the stopper, and end members at either end of the band, which are embedded in either side of the body of the stopper to as to secure the anchor in the stopper. More preferably, the anchor may be I-shaped. In a preferred arrangement, the proximal end of the guidewire is connected to the band of the anchor. Suitable means of connection include welding when, for example, the anchor is formed of metal and an epoxy resin when, for example, the anchor comprises a rigid polymeric material.

Preferably, the catheter further includes a drainage channel separate from the guidewire channel for the drainage of urine. The drainage channel may extend along the centre of the elongate body and the guidewire channel may extend parallel to the drainage channel along one side of the elongate body. Optionally, the guidewire channel may also act as an irrigation channel for the introduction of sterile irrigating solution into the bladder once the guidewire has been removed.

The lubrication port may comprise a side arm in fluid communication with the guidewire channel adjacent to the proximal end of the catheter. The lubrication port may be provided with a closure member for preventing drainage of urine through the lubrication port when the guidewire has been removed. Preferably, the closure member is a one-way valve.

Advantageously, the guidewire extends beyond the distal tip of the catheter when the stopper is coupled to the lubrication port. Preferably, the guidewire comprises a hydrophilic polymeric material.

The distal tip of the catheter may be open-ended so as to allow for drainage of urine from the bladder. Beneficially, this feature may also allow for the catheter to be used with known guidewires for the purpose of changing a long-term urethral catheter, particularly a suprapubic catheter. This makes the catheter changing procedure much safer and easier because the tract of the supra-pubic catheter cannot be lost. This is a frequent issue when inadequately trained clinical practitioners change suprapubic catheters.

Preferably, the elongate body of the catheter may be provided with at least one further drainage hole adjacent to the distal tip of the catheter so as to increase drainage efficiency. More preferably, the catheter may be provided with two drainage holes, which are located adjacent to the distal tip, one on either side of the catheter, so as to further increase drainage efficiency. Moreover, the presence of three drainage holes means that even if one or two of the holes become blocked, for example with a blood clot, drainage of urine can continue to take place through the other drainage hole.

The distal tip of the catheter is preferably formed at an oblique angle so as to help reduce tissue trauma as the catheter passes through the urethra. The oblique angle also provides a leading edge which helps the catheter to follow the guidewire in the bulbar and prostatic urethra.

The catheter may further comprise an inflatable balloon located adjacent to the distal tip of the catheter for retaining the catheter inside the bladder. In order to inflate the balloon, the catheter may include an inflation channel for introducing inflation fluid into the balloon.

The catheter may typically be made from a flexible, resilient and biocompatible polymeric material, such as polytetrafluoroethene (PTFE), polyethylene, polypropylene, polyurethane, polyvinyl chloride (PVC), latex or silicone. The inflatable balloon can be made from the same material as the catheter and may comprise a fine distensible additional layer of polymeric material attached to the elongate shaft.

To promote ease of insertion, withdrawal and positioning of the catheter, the catheter may optionally be coated with a hydrophilic lubricious polymer, such as polyurea, polyurethane, polyurethaneurea, polyglycols, polyvinyl pyrrolidone (PVP) or carboxylic acids, esters, salts and amides of poly(meth)acrylic acid, which becomes slippery in the presence of an aqueous fluid. Additionally or alternatively, a tube of lubricant such as Lidocaine/Aquagel or Aquagel alone can be injected into the urethra. To reduce and delay the occurrence of an infection, the catheter may also be coated with an anti-infective material such as a silver alloy or an antibiotic.

The size of the catheter may typically be between 10 French and 26 French in diameter and between 20 cm and 50 cm in length. A longer length catheter is usually required for males since the male urinary tract is longer than the female urinary tract.

Also described herein, but not forming part of the invention, is a method of urinary catheterisation, comprising:
lubricating a guidewire located in a channel extending through a catheter, the catheter having an elongate body provided with a distal tip, a proximal end and one or more channels extending therethrough, and the guidewire having a distal tip and a proximal end;
inserting the distal tip of the guidewire into the urethra and navigating the guidewire along the urethra and into the bladder;
advancing the catheter over the guidewire until the distal tip of the catheter is located in the bladder; and
removing the guidewire from the urethra and the catheter.

In a first aspect, the present invention resides in a device for urethral stricture dilatation comprising:
a dilator having an elongate body provided with a distal end tapered towards a distal tip, a proximal end and a guidewire channel extending therethrough;
a guidewire extending through the guidewire channel for guiding the dilator upon insertion; and
a lubrication port at the proximal end of the elongate body for introduction of a guidewire lubrication fluid into the guidewire channel;
wherein the guidewire is provided with a stopper for preventing the proximal end of the guidewire from passing through the guidewire channel towards the distal tip of the dilator and wherein the stopper is adapted to receive a syringe for introducing guidewire lubrication fluid into the guidewire channel when the stopper is coupled to the lubrication port;
and wherein the distal end of the dilator is curved towards the distal tip, and tapers from a diameter of between 14 French and 24 French to 2 French.

The distal end of the dilator may taper along a length of between 3 cm and 4 cm towards the distal tip. Preferably, the distal end may be curved at an angle between 30° and 45° relative to the elongate body.

The dilator may typically be made from a resilient and biocompatible polymeric material so that it can push through the scar tissue of a urethral stricture. The tapered distal end of the dilator may be made from a different material to the remainder of the elongate body. For example, the tapered distal end may be made from a material which provides it with greater flexibility, reduced flexibility, greater slipperiness or reduced slipperiness compared to the remainder of the elongate body. Alternatively, or in addition thereto, the tapered distal end may be made from a material whose physical properties vary depending on the surrounding temperature. For example, the material may be one that distends (swells) upon contact with the patient's body but contracts upon contact with cold water. To promote ease of insertion, withdrawal and positioning of the dilator, the dilator may optionally be coated with a hydrophilic lubricious polymer, such as polyurea, polyurethane, polyurethaneurea, polyglycols, polyvinyl pyrrolidone (PVP) or carboxylic acids, esters, salts and amides of poly(meth)acrylic acid, which becomes slippery in the presence of an aqueous fluid. Additionally or alternatively, lubricant such as Lidocaine/Aquagel or Aquagel alone can be injected into the urethra. To reduce and delay the occurrence of an infection, the dilator may also be coated with an anti-infective material such as a silver alloy or an antibiotic.

Described herein, but not forming part of the invention, is a method of urethral stricture dilatation, comprising:
lubricating a guidewire located in a guidewire channel extending through a dilator, the dilator having an elongate body provided with a distal end, tapered towards a distal tip, and a proximal end, and the guidewire having a distal tip and a proximal end;
inserting the distal tip of the guidewire into the urethra and navigating the guidewire along the urethra and into the bladder;
advancing the dilator over the guidewire until the distal tip of the dilator is located in the bladder; and
removing the guidewire and the dilator from the urethra.

Also described herein, but not forming part of the invention in itself, is a guidewire for use in a medical device, wherein the guidewire is provided with a stopper for preventing loss of the guidewire. Preferably, the stopper is adapted to receive a syringe for introducing guidewire lubrication fluid onto the guidewire.

Advantageously, the dilator of the present invention may be used with known guidewires, for example, those comprising a core wire coated with hydrophilic polymer.

Also described herein, but not forming part of the invention, is a urinary catheterisation kit comprising:
a catheter having an elongate body provided with a distal tip, a proximal end and one or more channels extending therethrough;
a guidewire extending through one of said one or more channels for guiding the catheter upon insertion;
a lubrication port at or towards the proximal end of the elongate body for introduction of a guidewire lubrication fluid into the channel having the guidewire extending therethrough; and
a sterile package in which the catheter and guidewire are supplied for use, the package adapted to be opened to allow injection of guidewire lubrication fluid through the lubrication port to lubricate the guidewire in the channel having the guidewire extending therethrough and that portion of the guidewire extending beyond the distal tip of the catheter.

The kit may further comprise an external sterile package for housing the sterile package containing the catheter and guidewire, wherein the external sterile package additionally houses at least one syringe. Preferably, the at least one syringe is pre-filled with guidewire lubrication fluid.

In a second aspect, the present invention resides in a urethral stricture dilatation kit comprising:
a dilator having an elongate body provided with a distal end tapered towards a distal tip, a proximal end and a guidewire channel extending therethrough;
a guidewire extending through the guidewire channel for guiding the dilator upon insertion;
a lubrication port at the proximal end of the elongate body for introduction of a guidewire lubrication fluid into the guidewire channel;
wherein the guidewire is provided with a stopper for preventing the proximal end of the guidewire from passing through the guidewire channel towards the distal tip of the dilator and wherein the stopper is adapted to receive a syringe for introducing guidewire lubrication fluid into the guidewire channel when the stopper is coupled to the lubrication port; and
wherein the distal end of the dilator is curved towards the distal tip, and tapers from a diameter of between 14 French and 24 French to 2 French; and
a sterile package in which the dilator and guidewire are supplied for use, the package adapted to be opened to allow injection of guidewire lubrication fluid through the lubrication port to lubricate the guidewire in the guidewire channel and that portion of the guidewire extending beyond the distal tip of the dilator.

The kit may further comprise an external sterile package for housing the sterile package containing the dilator and guidewire, wherein the external sterile package additionally houses a syringe. Preferably, the syringe is pre-filled with guidewire lubrication fluid.

The present invention has the advantage of simplifying and improving the safety of the dilatation procedure. The integration of the guidewire into the dilator facilitates placement of the dilator in the urethra and provides a convenient and safe product for use by a medical/clinical practitioner or patient.

The guidewire of the device/kit of the present invention is especially advantageous because the stopper prevents loss of the proximal end of the guidewire along the guidewire channel into the urethra or bladder. Moreover, when the stopper is capable of receiving a syringe filled with lubrication fluid, it enables the hydrophilic guidewire to be lubricated whilst it is located within the catheter/dilator. This helps to maintain the sterility of the device whilst it is prepared for use and makes preparation more convenient for the user. Also, the fact that the device is still retained inside its internal wrapping at the time of lubrication ensures that the guidewire is fully lubricated along its entire length.

A number of definitions are provided that will assist in the understanding of the invention.

The term 'catheter' as used herein denotes a hollow tube used to drain fluid from a body cavity, duct or vessel, or to distend a body passage, and the term 'catheterisation' denotes the operation of introducing a catheter into the body.

The term 'dilator' as used herein denotes an instrument for dilating or distending a body cavity, duct, vessel, canal or orifices, and the term 'dilatation' as used herein denotes the operation of introducing a dilator into the body.

### BRIEF DESCRIPTION OF THE DRAWINGS

In order that the invention may be more readily understood, reference will now be made, by way of example, to the accompanying drawings in which:
Figure 1 is a device for urinary catheterisation as described herein but not forming part of the present invention: (a) is a side view of an indwelling catheter having a drainage channel, a balloon inflation channel and a guidewire channel with guidewire in-situ; (b) is a cross-sectional view of the catheter taken along the line II-II; (c) is a cross-sectional view of the catheter taken along the line III-III.
Figure 2 is a guidewire having a stopper for use in all embodiments of the present invention: (a) is a perspective view of a guidewire having a T-shaped stopper; (b) is a perspective view of a guidewire having a cylindrical stopper; (c) is an end view of the stopper as shown in (a) and (b).
Figure 3 is a side view of a device for urinary catheterisation as described herein but not forming part of the present invention.
Figure 4 is a side view of a device for urethral stricture dilatation in an embodiment of the present invention.
Figure 5 is a top view of the sterile packaging for a device of all embodiments of the present invention.

### DETAILED DESCRIPTION

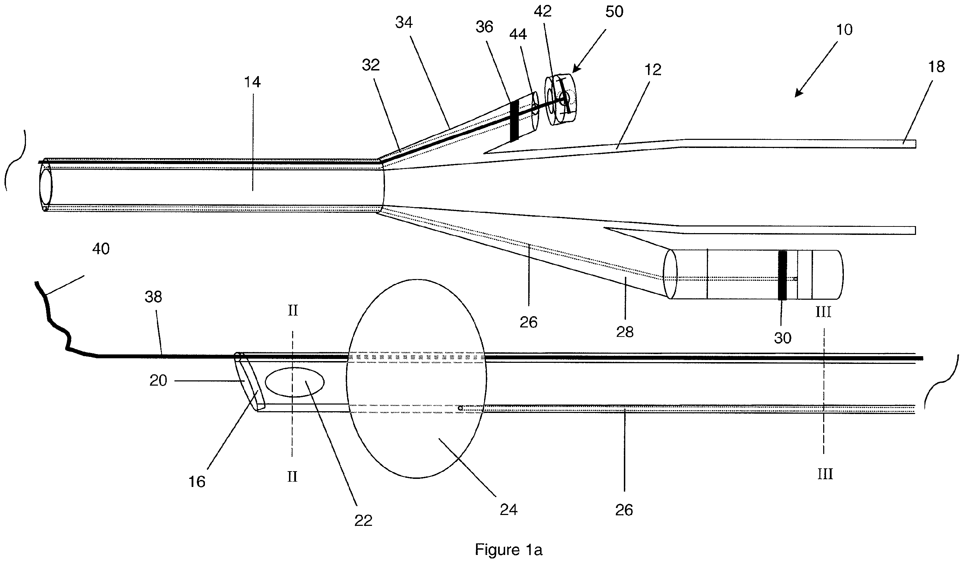

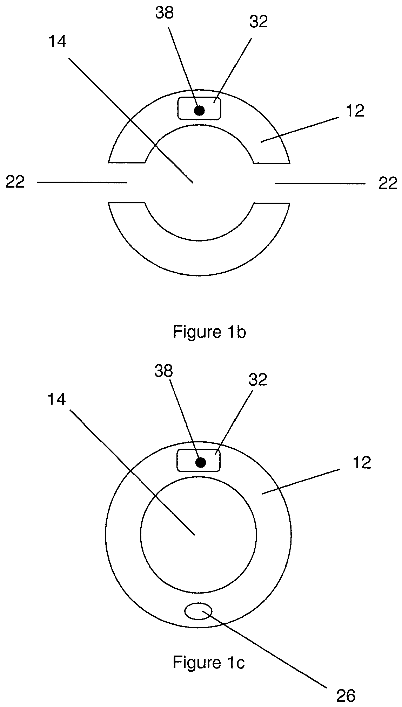

A device for urinary catheterisation is shown in Figure 1. The device comprises a catheter 10 having a flexible elongate shaft 12. The elongate shaft 12 defines a drainage channel 14 which extends along the length of the catheter 10 from the distal tip 16 of the catheter 10 to the proximal end 18 of the catheter 10. The proximal end 18 of the catheter 10 is capable of being connected to a fluid collector (not shown).

The distal tip 16 of the catheter is open-ended and formed at an oblique angle. The open end of the distal tip 16 defines a drainage hole 20, which allows fluid to enter the drainage channel 14. Two further drainage holes 22 are located adjacent to the distal tip 16, one on each side of the elongate shaft 12, to increase drainage efficiency of the device.

An inflatable balloon 24 is located adjacent to the distal tip 16 of the catheter 10 and encircles the elongate shaft 12. Typically, the balloon 24 has a maximum volume of between 10 ml and 30 ml and is spherical in shape, although various shapes may be used. The wall of the elongate shaft 12 houses an inflation channel 26, which extends along the length of the catheter 10 from the inflatable balloon 24 to an inflation channel side arm 28 positioned adjacent to the proximal end 18 of the catheter 10. The inflation channel 26 is cylindrical and typically has a diameter between 0.1 mm and 0.3 mm. The inflation channel side arm 28 houses a two-way valve 30 and is capable of receiving a syringe. The balloon 24 is inflated by inserting a syringe (not shown) filled with air, water or saline solution into the inflation channel side arm 28 and injecting the contents of the syringe into the inflation channel side arm 28, through the valve 30, and along the inflation channel 26 until the balloon 24 is sufficiently expanded. The valve 30 prevents the contents of the inflated balloon 24 from leaking out of the inflation channel side arm 28 until deflation is required. To deflate the balloon 24, an empty syringe is connected to the inflation channel side arm 28 and the contents of the balloon 24 are removed by suction.

The wall of the elongate shaft 12 further comprises a guidewire channel 32, which extends along the length of the catheter 10 from the distal tip 16 of the catheter 10 to a guidewire channel side arm 34 positioned adjacent to the proximal end 18 of the catheter 10 on the opposite side of the shaft to the inflation channel side arm 28. The guidewire channel 32 is oblong in cross-section although other shapes may be used, for example, oval, circular, or square in cross-section, and typically has dimensions of about 1-2 mm x 1mm. Both the guidewire channel 32 and the inflation channel 26 run parallel to the drainage channel 14 and are independent from the drainage channel 14. The guidewire channel side arm 34 has a one-way valve 36 to prevent urine from draining along the guidewire channel 32 and out of the opening 44 of the guidewire channel 32 when the guidewire 38 has been removed from the device. Alternative means of closing the guidewire channel 32 may be used.

A flexible guidewire 38 is located in the guidewire channel 32 and serves to guide the catheter 10 into the correct position upon insertion. The guidewire 38 is typically between 0.8 mm and 0.9 mm in diameter and between 100 cm and 180 cm in length, and extends from the proximal end of the guidewire channel side arm 34, along the full length of the guidewire channel 32 and beyond the distal tip 16 of the catheter 10.

The guidewire 38 comprises a core wire, typically made of stainless steel, platinum, shape memory alloys such as Nitinol, or other metal, which provides a degree of stiffness to the guidewire 38. The core wire extends almost the entire length of the guidewire 38, terminating shortly before the guidewire distal tip 40, and may taper in diameter towards the distal tip 40, thereby increasing the flexibility of the guidewire 38 towards the distal tip 40. The highly flexible distal tip 40, without the core wire, is typically between 3 cm and 9 cm in length. The flexibility of the distal tip 40 reduces tissue damage and degradation as the guidewire 38 enters the body and enables the user to safely and easily manoeuvre and navigate the guidewire 38 along the urinary tract towards the bladder.

The core wire of the guidewire 38 is coated with a hydrophilic polymer, such as polyurea, polyurethane, polyurethaneurea, polyglycols, polyvinyl pyrrolidone (PVP) or carboxylic acids, esters, salts and amides of poly(meth)acrylic acid. The hydrophilic coating becomes slippery in the presence of an aqueous fluid, such as water, and provides surface lubricity to the guidewire 38. This surface lubricity reduces friction between the guidewire 38 and body tissue as the guidewire 38 passes through the urinary tract and also reduces friction between the guidewire 38 and the guidewire channel 32 as the catheter 10 is passed over the guidewire 38.

The hydrophilic coating can comprise a simple coating of one polymer, a blending/complexing of two or more hydrophilic polymers, an interpenetrating network of polymers or one or more chemically reactive hydrophilic polymers.

A stopper 50 located at the proximal end 42 of the guidewire 38 prevents the guidewire 38 from disappearing along the guidewire channel 32 towards the distal tip 16 of the catheter 10 and becoming lost in the urethra or bladder.

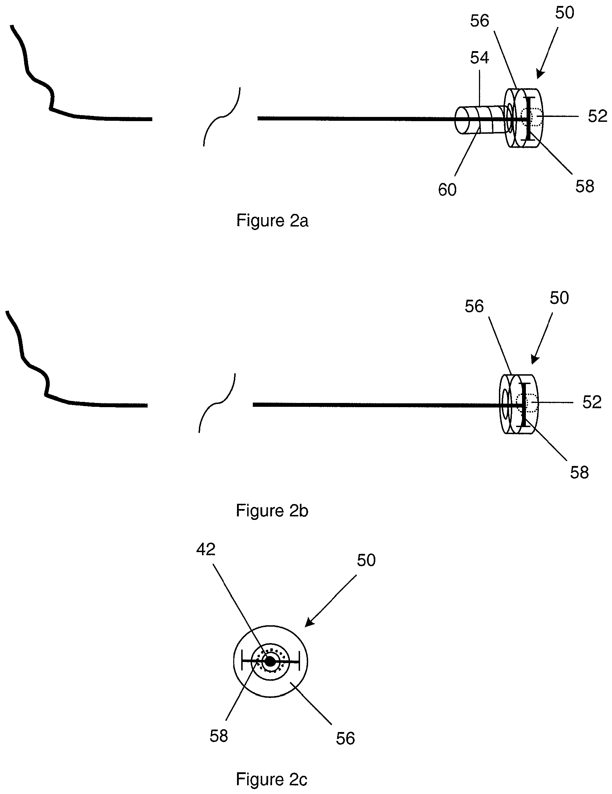

Figures 2(a) and 2(b) show two different stopper designs in more detail. Figure 2(a) shows a stopper 50 having a T-shaped configuration and comprising a hollow core 52 which extends through the body 54 and the head 56 of the stopper 50. The head 56 of the stopper 50 contains a narrow metal anchor 58 which traverses the hollow core 52 and is embedded in either side of the head 56 of the stopper 50. The proximal end 42 of the guidewire 38 is welded to the metal anchor 58, thereby securely connecting the guidewire 38 to the stopper 50.

The head 56 of the stopper 50 is capable of receiving a syringe (not shown), with the nose of the syringe fitting into the hollow core 52 of the stopper 50. To lubricate the guidewire 38, a syringe filled with aqueous fluid, such as water, is inserted into the head 56 of the stopper 50 and the fluid is injected into the stopper 50, through the guidewire channel side arm 34 and along the guidewire channel 32 towards the distal tip 16 of the catheter 10. The narrow width of the metal anchor 58 in the head 56 of the stopper 50 means that fluid can easily move past the anchor 58 as it passes through the stopper 50.

The body 54 of the stopper 50 is sized so as to be able to fit into the guidewire channel side arm 34, whilst the head 56 of the stopper 50 is larger than the opening 44 of the guidewire channel 32 and abuts the opening 44 so as to prevent the stopper 50 from passing through the guidewire channel 32 towards the distal tip 16 of the catheter 10. The stopper 50 is releasably connected to the guidewire channel side arm 34 by means of a screw thread 60 on the outer surface of the body 54 of the stopper 50 which interacts with a complimentary screw thread on the inner surface of the guidewire channel 32 to provide a secure connection between the guidewire 38 and the catheter 10 prior to and during use of the guidewire 38.

An alternative configuration of stopper is shown in Figure 2(b). In this instance, the stopper 50 comprises only a head 56 having a hollow core 52 into which the nose of a syringe fits. The head 56 of the stopper 50 is connected to the proximal end 42 of the guidewire 38 by means of an anchor 58, as previously described, and abuts the opening 44 of the guidewire channel 32 to prevent the stopper 50 from passing through the guidewire channel 32 towards the distal tip 16 of the catheter 10. To enable a secure connection between the stopper 50 and the catheter 10, the head 56 of the stopper 50 can be releasably coupled to the guidewire channel side arm 34 as previously described with reference to Figure 2(a). Figure 2(c) shows an end view of the stopper 50 of Figure 2(a) and 2(b) and the configuration of the anchor 58 traversing the hollow width 52 of the stopper 50.

In an exemplary use, the device as shown in Figure 1 is inserted into the patient's urinary tract and retained in the bladder to drain away urine in the following manner.

Firstly, the full length of guidewire 38 is lubricated by inserting a syringe filled with water into the stopper 50 located at the proximal end 42 of the guidewire 38 and injecting the contents of the syringe into the stopper 50, along the guidewire channel side arm 34 and along the full length of the guidewire channel 32. Upon contact with the water, the hydrophilic coating of the guidewire 38 swells and becomes slippery and provides surface lubricity to the guidewire 38. The surface lubricity helps to reduce friction between the guidewire 38, the guidewire channel 32 and body tissue as the guidewire 38 passes through the urinary tract. Once the guidewire 38 is fully lubricated, it is then ready to be inserted into the urethra of the patient.

A lubricant is inserted into the urethra of the patient, then the tip 40 of the guidewire 38 is inserted into the urethra and the guidewire 38 is navigated along the path of the urethra towards the bladder. The high flexibility of the guidewire tip 40 reduces tissue damage and degradation as the guidewire 38 moves along the urinary tract and helps the user to circumvent any obstructions in the urethra. By inserting the entire length of the guidewire 38 the user can be sure that the guidewire 38 has reached the bladder. The presence of the stopper 50 prevents the guidewire 38 from disappearing along the guidewire channel 32.

Once the guidewire 38 is fully inserted into the bladder, the catheter 10 is ready to be advanced over the guidewire 38. The surface lubricity of the guidewire 38 , combined with the optional surface lubricity of the catheter 10, reduces the frictional resistance between the guidewire 38 and the guidewire channel 32 as the catheter 10 slides over the guidewire 38. The guidewire 38 defines the path along which the catheter 10 travels and makes catheter insertion much safer and more reliable, thereby causing minimal discomfort to the patient.

The catheter 10 is in its fully inserted position when the distal tip 16 of the catheter 10 reaches the bladder and the balloon 24 is beyond the bladder neck. The user can assess whether or not the end of the catheter 10 is correctly positioned in the bladder by observing the drainage of urine through the drainage channel 14 of the catheter 10. If no urine drainage is observed, the distal tip 16 of the catheter 10 is still in the urethra and the catheter 10 will need to be advanced further along the guidewire 38. Once urine drainage is observed, the user can be sure that the catheter 10 is in the correct position and the guidewire 38 can safely be removed from the bladder and the catheter 10. The catheter 10 is then connected at its proximal end 18 to a fluid collector (not shown).

To retain the catheter 10 in the bladder, the balloon 24 is inflated by inserting a syringe filled with water or saline solution into the inflation channel side arm 28 and injecting the contents of the syringe into the inflation channel side arm 28 and along the inflation channel 26 until the balloon 24 is filled. The catheter 10 remains in place until the patient is able to void again after surgical treatment, until measurement of urine output is no longer required or until the catheter 10 needs to be replaced. To remove the catheter 10, the balloon 24 is deflated by connecting an empty syringe to the inflation channel side arm 28 and sucking the water or saline solution out of the balloon 24, and then the catheter 10 is carefully removed.

The guidewire channel 32 can optionally be used as an irrigation channel, once the catheter 10 has been fully inserted and the guidewire 38 removed, to flush sterile irrigating solution into the bladder. In this instance, an irrigation fluid connector (not shown) is attached to the guidewire channel side arm 34 using a releasable connection mechanism such as a screw fit, a push-fit, a snap-lock, a twist-lock or a Luer Lock. Once the bladder has been suitably irrigated and cleared of blood clots etc., the irrigation fluid connector is removed.

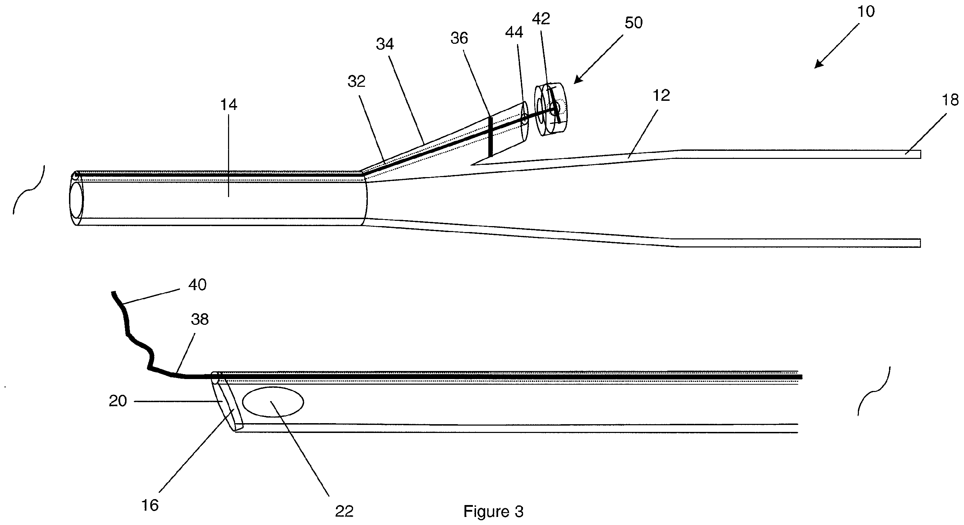

A second example of a urinary catheterisation device is shown in Figure 3. This device is intended for patients who require intermittent catheterisation in order to empty their bladder because of bladder failure or after bladder reconstruction. The device does not need to be retained in the bladder so it does not have an inflatable balloon but it is otherwise identical to the first exemplified catheterisation device. In this instance, the catheter 10 is inserted into the patient's bladder and retained there only until complete drainage of urine from the bladder has been observed.

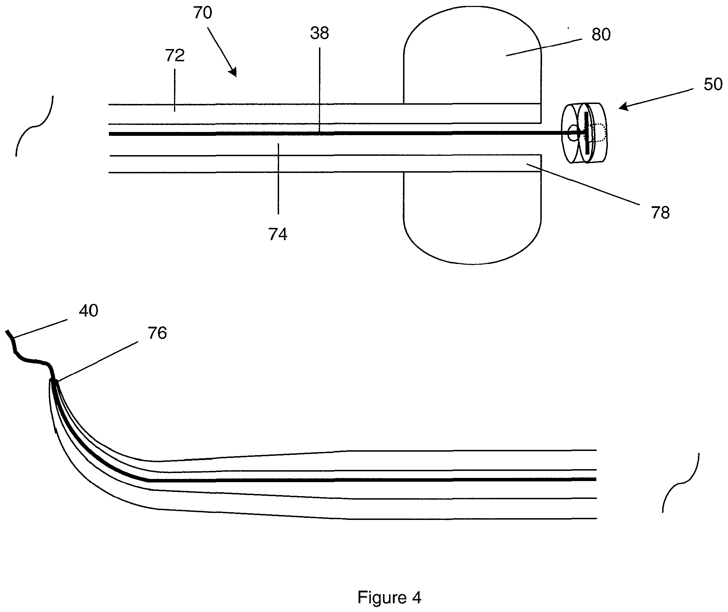

An embodiment of the invention is shown in Figure 4. This device is for dilating urethral strictures and comprises a dilator 70 having an elongate shaft 72. The elongate shaft 72 defines a guidewire channel 74 which extends along the length of the dilator 70 from the distal tip 76 of the dilator 70 to the proximal end 78 of the dilator 70. The guidewire channel 74 is cylindrical and typically has a diameter between 0.8 mm and 1.0 mm, although it tapers at the distal end 76 of the elongate shaft 72 towards the distal tip 76. Both the distal tip 76 and the proximal end 78 of the guidewire channel 74 are open-ended and the proximal end 78 is provided with a handle 80 for ease of use.

A flexible guidewire 38 is located in the guidewire channel 74 and serves to guide the dilator 70 into the correct position upon insertion. The guidewire 38 and stopper 50 of this embodiment of the invention is identical to that used in the first and second examples of the catheterisation device. As per the previous examples, the stopper 50 located at the proximal end 42 of the guidewire 38 prevents the guidewire 38 from disappearing along the guidewire channel 74, except in this embodiment there is no guidewire channel side arm so the stopper 50 interacts with the proximal end 78 of the dilator 70 rather than the proximal end of the guidewire channel side arm.

The size of the dilator 70 is typically between 2-14 French and 2-24 French in diameter and between 30 cm and 40 cm in length. The distal end 77 of the dilator 70 is curved at an angle of approximately 30-45° to help follow the natural curve of the urethra, although straight tips may also be used. Moreover, the distal end 77 is tapered over a length of approximately 4 cm towards the distal tip 76 from a diameter of between 14 French and 24 French at the widest point of the elongate shaft 72 down to 2 French at the distal tip 76. The dilator 70 also has circumferential markings (not shown) every 10 cm along the length of the elongate shaft 72 in order to assist the medical/clinical practitioner in assessing the length of dilator 70 that has been inserted into the urethra. The close fit of the distal tip 76 around the guidewire 38 helps to prevent urethral tissue from becoming caught between the distal tip 76 and the guidewire 38 and therefore reduces the likelihood of tissue damage. Furthermore, the tapered distal end 77 and the narrow diameter of the distal tip 76 causes the distal end 77 of the dilator to be more flexible than the remainder of the wider elongate shaft 72.

The relatively long tapered distal end 77 of the dilator 70 of the present invention compared to known dilators allows for a large increase in diameter in a single dilator. This enables the user to dilate the stricture up to the maximum width required using a single dilator rather than having to use multiple dilators each having a slightly increased diameter (e.g. 6/10F, 8/12F, 12/14F, 14/18F, etc. whereby the first number denotes the calibre of the tip and the second number denotes the calibre of the shaft). Using one dilator safely over a guidewire speeds up the dilatation procedure and reduces the number of dilators normally used to treat a urethral stricture and hence the amount of waste material produced.

In exemplary use, the device of the invention, as shown in Figure 4 is inserted into the patient's urinary tract to dilate a urethral stricture in the following manner.

Firstly, the full length of the guidewire 38 is lubricated and fully inserted into the urethra as per the first and second examples of the catheterisation device. The highly flexible tip 40 of the guidewire 38 helps the user to find the lumen of the urethral stricture. Once the guidewire 38 has been advanced into the bladder, the dilator 70 is ready to be advanced over the guidewire 38. The dilator 70 is inserted until the distal tip 76 reaches the bladder thereby stretching the stricture to the calibre of the dilator shaft. The dilator 70 and the guidewire 38 are then removed from the urethra.

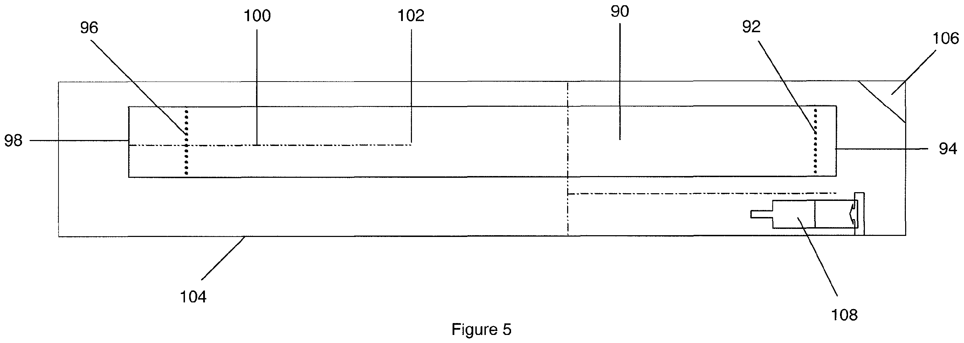

Figure 5 shows how the device of the invention is packaged in a sterile environment ready for use. This packaging can be used for all exemplary catheters, i.e. an indwelling catheter (Figure 1), an intermittent catheter (Figure 3) or a dilator of the invention (Figure 4), having an integral guidewire 38. The packaging enables the device to be prepared for use in a convenient and sterile manner.

The device of the invention (not shown) is contained within a sterile internal wrapping 90, which is typically made from plastic. The internal wrapping 90 has a top side and a bottom side (not shown) and is substantially rectangular in shape for ease of manufacture, though other shapes are equally suitable. A first perforation 92 is located at the proximal end 94 of the internal wrapping 90, which is where the proximal end of the device is situated. A second perforation 96 is located at the distal end 98 of the internal wrapping 90, which is where the distal tip of the device is situated. Both the first and second perforations extend across the full width of the top and bottom side of the internal wrapping 90, enabling the internal wrapping 90 to be completely torn at these positions. A third perforation 100 extends along the top side of the internal wrapping 90 from the distal end 98 to approximately the mid-point 102 of the wrapping 90.

The internal wrapping 90 is housed within a sterile external wrapping 104, which is typically made of plastic or paper. The external wrapping 104 has a top side, a bottom side (not shown) and is substantially rectangular in shape, and comprises a peelable corner 106 at its proximal end. Additionally housed within the external wrapping 104 is a syringe 108 pre-filled with water for lubricating the guidewire 38. Where the device comprises an indwelling catheter 10 with an integral guidewire 38, the external wrapping 104 houses two syringes filled with water; one for lubricating the guidewire and one for inflating the balloon. Alternatively, the syringes within the external wrapping 104 may be empty and can be filled by the user when the device is ready to be used.

The device of the invention is prepared for use in the following manner. First, the external wrapping 104 is opened by pulling at the peelable corner 106 at the proximal end of the wrapping. The contents of the external wrapping 104, which include the internal wrapping 90 containing the device and at least one syringe 108 filled with water, are then transferred onto a sterile surface, such as a table or the internal sterile side of the external wrapping.

Next, the first perforation at the proximal end 92 of the internal wrapping 92 is torn to expose the proximal end of the device. The syringe 108 filled with water is then inserted into the stopper 50 located at the proximal end of the guidewire 38 of the device and the water is injected through the stopper 50 in order to lubricate the guidewire 38. The entire length of the guidewire 38 becomes lubricated by virtue of the fact that it is curled up within the internal wrapping 90 and becomes immersed in the water.

Once the guidewire 38 has been fully lubricated, the second and third perforations 96, 100 of the internal wrapping 90 are torn to expose the distal tip of the device. The internal wrapping 90 can then be discarded or it can be kept in position until after the guidewire 38 has been inserted in order to further protect the exemplary catheter or inventive dilator.

Although particular embodiments of the invention have been disclosed herein in detail, this has been done by way of example and for the purposes of illustration only.

## Claims

1. A device for urethral stricture dilatation comprising:
a dilator having an elongate body provided with a distal end tapered towards a distal tip, a proximal end and a guidewire channel extending therethrough;
a guidewire extending through the guidewire channel for guiding the dilator upon insertion; and
a lubrication port at the proximal end of the elongate body for introduction of a guidewire lubrication fluid into the guidewire channel;
wherein the guidewire is provided with a stopper for preventing the proximal end of the guidewire from passing through the guidewire channel towards the distal tip of the dilator and wherein the stopper is adapted to receive a syringe for introducing guidewire lubrication fluid into the guidewire channel when the stopper is coupled to the lubrication port;
and wherein the distal end of the dilator is curved towards the distal tip, and tapers from a diameter of between 14 French and 24 French to 2 French.

2. The device according to claim 1, wherein:
i) the stopper comprises a head portion and a body portion arranged in a T-shaped configuration and having a hollow core extending therethrough, the body portion capable of being received in the lubrication port and the head portion capable of receiving the syringe; or
ii) the stopper comprises a head portion having a hollow core extending therethrough, the head portion capable of receiving the syringe.

3. The device according to claim 2, wherein the head portion is larger than the opening of the lubrication port.

4. The device according to any preceding claim, wherein:
i) the stopper is capable of being releasably connected to the lubrication port; or
ii) the stopper is capable of being releasably connected to the lubrication port and the stopper is releasably connected to the lubrication port by a screw means, a push-fit means or a twist-lock means.

5. The device according to any preceding claim, wherein the stopper is provided with an anchor for connecting the proximal end of the guidewire to the stopper, the anchor being configured so as to allow the guidewire lubrication fluid to flow around the anchor as it passes through the stopper and into the guidewire channel.

6. The device according to claim 5 as dependent on any of claims 2 to 4, wherein the anchor comprises a band, which traverses the width of the hollow core of the stopper, and end members at either end of the band, which are embedded in either side of the stopper to secure the anchor in the stopper.

7. The device according to claim 5 or claim 6, wherein the anchor is I-shaped.

8. The device according to claim 6 or claim 7, wherein the proximal end of the guidewire is connected to the band of the anchor.

9. The device according to any preceding claim, wherein the distal end of the dilator is tapered along a length of between 3 cm and 4 cm towards the distal tip.

10. The device according to any preceding claim, wherein the distal end is curved at an angle of 30° to 45° relative to the elongate body.

11. The device according to any preceding claim, wherein the guidewire extends beyond the distal tip of the dilator when the stopper is coupled to the lubrication port.

12. The device according to any preceding claim, wherein the guidewire comprises a hydrophilic polymeric material.

13. A urethral stricture dilatation kit comprising:
a device according to any of claims 1 to 12; ; and
a sterile package in which the dilator and guidewire are supplied for use, the package adapted to be opened to allow injection of guidewire lubrication fluid through the lubrication port to lubricate the guidewire in the guidewire channel and that portion of the guidewire extending beyond the distal tip of the dilator.

14. The kit according to claim 13, further comprising an external sterile package for housing the sterile package containing the dilator and guidewire, wherein the external sterile package additionally houses a syringe.

15. The kit according to claim 14, wherein the syringe is pre-filled with guidewire lubrication fluid.

## Patentansprüche

1. Vorrichtung für eine Dilatation einer Harnröhrenverengung, die Folgendes umfasst:
einen Dilator, der einen länglichen Körper aufweist, der mit einem distalen Ende, das sich in Richtung einer distalen Spitze verjüngt, einem proximalen Ende und einem Führungsdrahtkanal versehen ist, der sich dahindurch erstreckt;
einen Führungsdraht, der sich durch den Führungsdrahtkanal zum Führen des Dilators bei einem Einsetzen erstreckt; und
einen Gleitmittelzufuhranschluss an dem proximalen Ende des länglichen Körpers für ein Einführen eines Führungsdrahtgleitmittelfluids in den Führungsdrahtkanal;
wobei der Führungsdraht mit einem Verschlussstück zum Verhindern des proximalen Endes des Führungsdrahts, durch den Führungsdrahtkanal in Richtung der distalen Spitze des Dilators zu verlaufen, versehen ist, und wobei das Verschlussstück angepasst ist, um eine Spritze zum Einführen des Führungsdrahtgleitmittelfluids in den Führungsdrahtkanal aufzunehmen, wenn das Verschlussstück mit dem Gleitmittelzufuhranschluss gekoppelt ist;
und wobei das distale Ende des Dilators in Richtung der distalen Spitze gekrümmt ist und sich von einem Durchmesser zwischen 14 Charriere und 24 Charriere auf 2 Charriere verjüngt.

2. Vorrichtung nach Anspruch 1, wobei:
i) das Verschlussstück einen Kopfabschnitt und einen Körperabschnitt umfasst, die in einer T-förmigen Konfiguration angeordnet sind und einen hohlen Kern aufweisen, der sich dahindurch erstreckt, wobei der Körperabschnitt in der Lage ist, in dem Gleitmittelzufuhranschluss aufgenommen zu werden und der Kopfabschnitt in der Lage ist, die Spritze aufzunehmen; oder
ii) das Verschlussstück einen Kopfabschnitt umfasst, der einen hohlen Kern aufweist, der sich dahindurch erstreckt, wobei der Kopfabschnitt in der Lage ist, die Spritze aufzunehmen.

3. Vorrichtung nach Anspruch 2, wobei der Kopfabschnitt größer als die Öffnung des Gleitmittelzufuhranschlusses ist.

4. Vorrichtung nach einem der vorhergehenden Ansprüche, wobei:
i) das Verschlussstück in der Lage ist, mit dem Gleitmittelzufuhranschluss lösbar verbunden zu sein; oder
ii) das Verschlussstück in der Lage ist, mit dem Gleitmittelzufuhranschluss lösbar verbunden zu sein und das Verschlussstück mit dem Gleitmittelzufuhranschluss durch ein Schraubmittel, ein Steckmittel oder ein Drehverschlussmittel lösbar verbunden ist.

5. Vorrichtung nach einem der vorhergehenden Ansprüche, wobei das Verschlussstück mit einem Anker zum Verbinden des proximalen Endes des Führungsdrahts mit dem Verschlussstück versehen ist, wobei der Anker konfiguriert ist, um es dem Führungsdrahtgleitmittelfluid zu ermöglichen, um den Anker herum zu fließen, wenn es durch das Verschlussstück und in den Führungsdrahtkanal verläuft.

6. Vorrichtung nach Anspruch 5, in Abhängigkeit von einem der Ansprüche 2 bis 4, wobei der Anker ein Band, das die Breite eines hohlen Kerns des Verschlussstücks überquert, und Endelemente an beiden Enden des Bands umfasst, die in beiden Seiten des Verschlussstücks eingebettet sind, um den Anker in dem Verschlussstück zu sichern.

7. Vorrichtung nach Anspruch 5 oder 6, wobei der Anker L-förmig ist.

8. Vorrichtung nach Anspruch 6 oder 7, wobei das proximale Ende des Führungsdrahts mit dem Band des Ankers verbunden ist.

9. Vorrichtung nach einem der vorhergehenden Ansprüche, wobei das distale Ende des Dilators entlang einer Länge zwischen 3 cm und 4 cm in Richtung der distalen Spitze verjüngt ist.

10. Vorrichtung nach einem der vorhergehenden Ansprüche, wobei das distale Ende in einem Winkel von 30° bis 45° relativ zu dem länglichen Körper gekrümmt ist.

11. Vorrichtung nach einem der vorhergehenden Ansprüche, wobei sich der Führungsdraht über die distale Spitze des Dilators hinaus erstreckt, wenn das Verschlussstück mit dem Gleitmittelzufuhranschluss gekoppelt ist.

12. Vorrichtung nach einem der vorhergehenden Ansprüche, wobei der Führungsdraht ein hydrophiles Polymermaterial umfasst.

13. Kit für die Dilatation einer Harnröhrenverengung, das Folgendes umfasst:
eine Vorrichtung nach einem der Ansprüche 1 bis 12; und
eine sterile Verpackung, in der der Dilator und der Führungsdraht zur Verwendung bereitgestellt werden, wobei die Verpackung angepasst ist, um geöffnet zu werden, um eine Injektion des Führungsdrahtgleitmittelfluids durch den Gleitmittelzufuhranschluss zu ermöglichen, um den Führungsdraht in dem Führungsdrahtkanal und jenen Abschnitt des Führungsdrahts gleitfähig zu machen, der sich über die distale Spitze des Dilators hinaus erstreckt.

14. Kit nach Anspruch 13, das ferner eine äußere sterile Verpackung zum Unterbringen der sterilen Verpackung umfasst, die den Dilator und den Führungsdraht enthält, wobei die äußere sterile Verpackung zusätzlich eine Spritze unterbringt.

15. Kit nach Anspruch 14, wobei die Spritze mit Führungsdrahtgleitmittelfluid vorgefüllt ist.

## Revendications

1. Dispositif de dilatation de sténose urétrale comprenant :
un dilatateur ayant un corps allongé pourvu d'une extrémité distale rétrécissant vers une pointe distale, une extrémité proximale et un canal de fil-guide s'étendant à travers celui-ci ;
un fil-guide s'étendant à travers le canal de fil-guide permettant de guider le dilatateur lors de l'insertion ; et
un orifice de lubrification à l'extrémité proximale du corps allongé pour l'introduction d'un fluide de lubrification de fil-guide dans le canal de fil-guide ;
le fil-guide étant pourvu d'un obturateur pour empêcher l'extrémité proximale du fil-guide de passer à travers le canal de fil-guide vers la pointe distale du dilatateur et l'obturateur est adapté pour recevoir une seringue permettant d'introduire le fluide de lubrification du fil-guide dans le canal de fil-guide lorsque l'obturateur est accouplé à l'orifice de lubrification ;
et l'extrémité distale du dilatateur étant incurvée vers la pointe distale, et rétrécissant depuis un diamètre compris entre 14 French et 24 French à 2 French.

2. Dispositif selon la revendication 1, dans lequel :
i) l'obturateur comprend une partie tête et une partie corps agencées dans une configuration en forme de T et ayant un noyau creux s'étendant à travers celles-ci, la partie corps pouvant être reçue dans l'orifice de lubrification et la partie tête pouvant recevoir la seringue ; ou
ii) l'obturateur comprend une partie tête ayant un noyau creux s'étendant à travers celui-ci, la partie tête pouvant de recevoir la seringue.

3. Dispositif selon la revendication 2, dans lequel la partie tête est plus grande que l'ouverture de l'orifice de lubrification.

4. Procédé selon l'une quelconque des revendications précédentes, dans lequel :
i) l'obturateur peut être relié de manière amovible à l'orifice de lubrification ;
ii) l'obturateur peut être relié de manière amovible à l'orifice de lubrification et l'obturateur est relié de manière amovible à l'orifice de lubrification par un moyen à vis, un moyen à ajustement légèrement dur ou un moyen de verrou rotatif.

5. Dispositif selon l'une quelconque des revendications suivantes, dans lequel l'obturateur est pourvu d'un point d'ancrage permettant de relier l'extrémité proximale du fil-guide à l'obturateur, le point d'ancrage étant conçu de manière à permettre au fluide de lubrification du fil-guide de s'écouler autour du point d'ancrage lors de son passage à travers l'obturateur et dans le canal de fil-guide.

6. Dispositif selon la revendication 5 dépendant de l'une quelconque des revendications 2 à 4, dans lequel le point d'ancrage comprend une bande qui traverse la largeur d'un noyau creux de l'obturateur, et des éléments d'extrémité au niveau de chaque extrémité de la bande, qui sont intégrés de chaque côté de l'obturateur afin de fixer le point d'ancrage dans l'obturateur.

7. Dispositif selon la revendication 5 ou la revendication 6, dans lequel le point d'ancrage est en forme de I.

8. Dispositif selon la revendication 6 ou 7, dans lequel l'extrémité proximale du fil-guide est reliée à la bande du point d'ancrage.

9. Dispositif selon l'une quelconque des revendications précédentes, dans lequel l'extrémité distale du dilatateur rétrécit le long d'une longueur comprise entre 3 cm et 4 cm vers la pointe distale.

10. Dispositif selon l'une quelconque des revendications précédentes, dans lequel l'extrémité distale est incurvée à un angle de 30° à 45° par rapport au corps allongé.

11. Dispositif selon l'une quelconque des revendications précédentes, dans lequel le fil-guide s'étend au-delà de la pointe distale du dilatateur lorsque l'obturateur est accouplé à l'orifice de lubrification.

12. Dispositif selon l'une quelconque des revendications précédentes, dans lequel le fil-guide comprend un matériau polymère hydrophile.

13. Kit de dilatation de sténose urétrale comprenant :
un dispositif selon l'une quelconque des revendications 1 à 12 ; et
un emballage stérile dans lequel le cathéter et le fil-guide sont fournis pour une utilisation, l'emballage étant adapté pour être ouvert afin de permettre l'injection de fluide de lubrification de fil-guide à travers l'orifice de lubrification afin de lubrifier le fil-guide dans le canal de fil-guide et cette partie du fil-guide s'étendant au-delà de l'extrémité distale du cathéter.

14. Kit selon la revendication 13, comprenant en outre un emballage stérile externe pour loger l'emballage stérile contenant le dilatateur et le fil-guide, l'emballage stérile externe logeant en outre une seringue.

15. Kit selon la revendication 14, dans lequel la seringue est pré-remplie de fluide de lubrification de fil-guide.
